(19) Europäisches Patentamt
European Patent Office
Office européen des brevets

(11) Publication number: **0 346 865 A2**

# (12) EUROPEAN PATENT APPLICATION

(21) Application number: 89110788.0

(51) Int. Cl.4: **C12N 11/02 , C12P 19/26**

(22) Date of filing: **14.06.89**

(30) Priority: **16.06.88 US 208194**

(43) Date of publication of application: **20.12.89 Bulletin 89/51**

(84) Designated Contracting States: **AT BE CH DE ES FR GB GR IT LI LU NL SE**

(71) Applicant: **E.I. DU PONT DE NEMOURS AND COMPANY (a Delaware corporation)**
**1007 Market Street**
**Wilmington Delaware 19898(US)**

(72) Inventor: **Moran, John Richard**
**27 Dogwood Drive**
**Kennett Square Pennsylvania 19348(US)**

(74) Representative: **von Kreisler, Alek, Dipl.-Chem. et al**
**Patentanwälte Von Kreisler-Selting-Werner**
**Deichmannhaus am Hauptbahnhof**
**D-5000 Köln 1(DE)**

## (54) Polynucleotide phosphorylase immobilized on epoxy-activated beads.

(57) Polynucleotide phosphorylase immobilized by covalent bonding to particles of a water-insoluble, epoxy-activated organic polymer is used to catalyze synthesis of polyribonucleotides from nucleotide diphosphates or production of nucleotide diphosphates by phosphorolysis of polyribonucleotides.

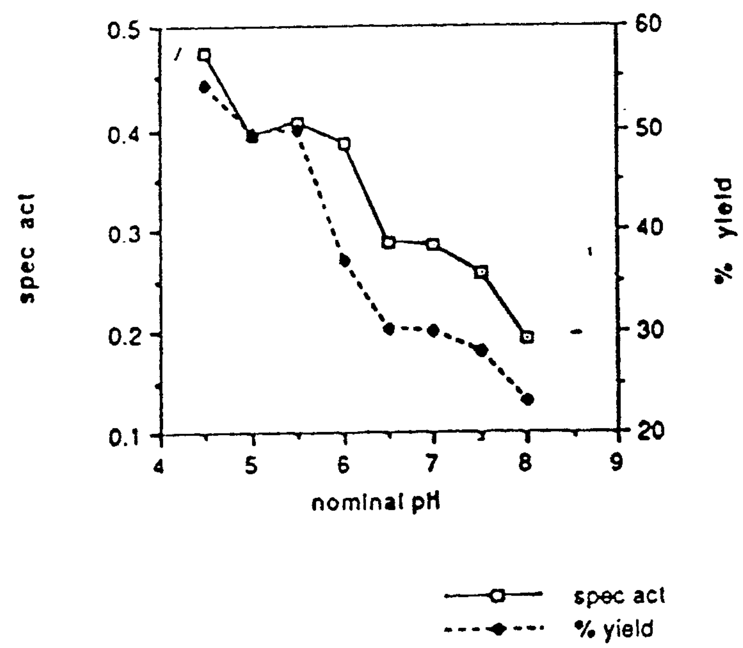


FIG. 1

EP 0 346 865 A2

Xerox Copy Centre

# POLYNUCLEOTIDE PHOSPHORYLASE IMMOBILIZED ON EPOXY-ACTIVATED BEADS

## FIELD OF THE INVENTION

This invention relates to immobilized polynucleotide phosphorylase (PNPase).

## BACKGROUND OF THE INVENTION

Polynucleotide phoshorylase is a bacterial enzyme which catalyzes the synthesis of single strand polyribonucleotide (also called polyribonucleoside monophosphate, poly NMP or NMPn) from ribonucleotide diphosphates (NDP's) in the presence of magnesium ions:

$NDP + (NMP)_{n-1} \text{<---->} (NMP)_n + P_i$

where n is an integer and $P_i$ represents phosphate ion. Each NDP is added sequentially to the free 3'-hydroxyl terminus of previously formed polyribonucleotide $(NMP)_{n-1}$. Frequently, a primer strand of $(NMP)_{n-1}$ is used at the beginning of the reaction. Reaction equilibrium can be shifted in the direction of breakdown of polyribonucleotide by increasing the phosphate ion concentration. Thus, PNPase can be used to catalyze the phosphorolysis of polyribonucleotides to produce ribonucleoside diphosphates as well as to catalyze the production of polyribonucleotides from ribonucleoside diphosphates.

The poly NMP can be a naturally occurring single strand ribonucleic acid (ssRNA) or a homopolymer or copolymer ssRNA such as polyinosinic acid (poly I), polycytidylic acid (poly C) or polycytidylic/uridylic acid (poly C,U). Poly I and poly C or poly C,U can be annealed to form double stranded ribonucleic acid (dsRNA) useful for induction of interferon and in the treatment of viral infections and cancer, as disclosed, for example in U.S. patent 4,130,641 and European Published Applications 113,162 and 213,921.

The synthesis and phosphorolysis reactions are normally carried out with the PNPase in solution as the free enzyme. However, as described in Boyer, Editor, The Enzymes, 3d Edition, volume XV, part 8, (1982), page 546, PNPase has been immobilized on various insoluble materials such as nitrocellulose filters, cellulose beads, mercerized cellulose, Sepharose® 4B polysaccharide, hydrazide agarose, diazotized p-aminobenzenesulfonylethyl (ABSE) agarose, ABSE Sephadex® G-200 and ABSE cellulose. Boyer states that the immobilized PNPase has advantages over the soluble enzyme; yield of polymerization is improved with some supports under pH conditions which favor polymerization over phosphorolysis, separation of reaction products from enzyme is simplified, and the enzyme can be recycled multiple times. Thang et al., Biochemical and Biophysical Research Communications, Vol. 31, pp. 1-8 (1968) discloses PNPase immobilized on a nitrocellulose filter. Hoffman et al., Biochemical and Biophysical Research Communications, Vol. 41, No. 3, pp 710-714 (1970) discloses use of PNPase bound to CNBr activated cellulose for production of homopolynucleotides. Smith et al., FEBS Letters, Vol. 30, No. 2, pp. 246-248 (1973) discloses use of PNPase bound to mercerized cellulose or Sepharose® or aminoalkylsilane glass beads for preparation of single strand polyribonucleotides. Soreq et al., Journal of Biological Chemistry, Vol. 252, pp.6885-6888 (1977), discloses PNPase immobilized onto CNBr activated Sepharose® for phosphorolysis of polynucleotides. Bachner et al., Biochemical and Biophysical Research Communications, Vol. 63, No. 2, pp. 476-483 (1975) mentions that polynucleotides were synthesized with PNPase immobilized on agarose. Yamauchi et al., Journal of Fermentation Technology, Vol. 64, No. 6, pp. 517-522(1986) and Yamauchi et al., Journal of Fermentation Technology, Vol. 64, No. 5, pp . 455-457 (1985) disclose PNPase immobilized on aminopropyl porous glass by various covalent bonding methods, and states that the product obtained by means of glutaraldehyde bonding is suitable for practical production of polynucleotides such as poly I and poly C. Yamauchi also refers to prior methods of immobilizing PNPase on CNBr activated cellulose and on silica gel, but states that these methods were not entirely satisfactory. Brentnall et al., Tetrahedron Letters, No. 25, pp. 2595-2596 (1982) discloses polymerization of 8-chloroadenosine diphosphate using PNPase immobilized on cellulose. Vang et al., Biochemical and Biophysical Research Communications, Vol. 90, No. 2, pp. 606-614 (1979) discloses immobilization of PNPase on CNBr activated Sepharose® and states that the bound enzyme retains 70% of its activity in polymerization of NDP's. Cashion et al., Biotechnology and Bioengineering, Vol. XXIV, pp. 493-423 (1982), Cashion et al., Nucleic Acids Research, Symposium Series No. 7, pp 173-189 (1980), and Cashion et al. U.S. patent 4,379,843 describe the immoblization of PNPase and other enzymes on tritylated agarose or Sepharose® beads by non-covalent bonding. Ansberga et al., U.S.S.R. published application 810,717 discloses PNPase immobilized in gamma aluminum hydroxide for

synthesis of polynucleotides. Kratasyuk et al., Izv. Sib. Otd Akad. SSSR. Ser. Biol. Nauk. Vol. 2, pp. 93-99 (1978) discloses PNPase immobilized on a macroporous matrix for synthesis and phosphorolysis of polynucleotides. Kumarev et al., Bioorg. Khim., Vol. 2, pp. 700-707 (1976) discloses immobilization of PNPase on a propionaldehyde-containing silochrome support. Broom, A.D., in an unpublished personal communication, describes polynucleotide synthesis experiments using PNPase immobilized on a number of different supports, including derivatized cellulose, polyacrylamide, and agarose; the commercially available support which gave best results was Affi-Gel®10 (Bio-Rad), a succinimide derivative of agarose, but even better results were obtained with a benzyl derivative of sepharose®4B, which bound PNPase hydrophobically rather than covalently.

Epoxy-activated particles, i.e. particles of polymers that contain oxirane groups as active protein-binding groups, have been used for immobilization of various enzymes, but have not been used for immobilization of PNPase. Kraemer et al. US patents 4,070,348, 4,190,713, 4,208,309, 4,247,643 and 4,511,694 disclose particles of this type and their use to bind enzymes. The Kraemer et al. particles are composed of copolymers of (A) an ethylenically unsaturated monomer having a glycidyl group, (B) a comonomer having at least two radical polymerizable double bonds, and (C) a radical-polymerizable water soluble comonomer. Röhm Pharma GmbH markets a family of polymer particles of the type taught in the Kramer patents under the trademark Eupergit® C. Röhm Pharma's trade literature describes the particles as oxirane acrylic beads made by copolymerization of methacrylamide, N,N′-methylene-bis-methacrylamide, glycidyl methacrylate and/or allyl glycidyl ether, and states that enzymes can be immobilized on the particles in high yields (60-90% of the activity of the free enzymes). Röhm Pharma recommends that under standard conditions protein immobilization be carried out at pH 7-8, but recommends acidic conditions for binding some proteins such as pepsin and indicates that the optimal pH for binding any given enzyme can be selected from a wide range (pH 0-12). Eupergit® C is available in 4 variations which differ in particle size and porosity. Eupergit® C, C 30 N, and C 250 L are macroporous beads with average diameters of approximately 150, 30 and 250 µm, respectively, and pores (channels and cavities) approximately 0.03, 0.04 and 0.15 µm in diameter, respectively. Eupergit® C 1 Z is a non-porous microbead approximately 1 µm in average diameter.

Other companies also offer epoxy-activated polymer particles for immobilization of enzymes. Pharmacia Laboratories offers epoxy-activated Sepharose® 6B. Riedel-de Haën offers VA-epoxy BIOSYNTH® beads of vinyl acetate and divinylethylene-urea with a surface modified by hydrolysis of the acetate groups with oxirane groups. The VA-epoxy beads are about 50-200 µm in diameter and contain pores about 0.03 µm in diameter.

Matthews U.S. patents 3,844,892 and 3,932,557 disclose immobilization of enzymes on epoxy-containing copolymers prepared by reacting a monomer containing a 1,2-epoxy group and terminal unsaturation with an olefin monomer such as acrylonitrile, methacrylonitrile, methyl acrylate or methyl methacrylate. The first monomer is made by reacting a diepoxide such as a diglycidyl ether of bisphenol A with a difunctional olefin such as acrylic acid or methacrylic acid.

Bigwood U.S. patents 4,582,860 and 4,612,288 disclose immobilization of enzymes on epoxy-activated polymer beads made by copolymerizing an oxirane-bearing monovinyl monomer, such as glycidyl acrylate or methacrylate or allyl glycidyl ether, with a trivinyl monomer such as trimethylolpropane trimethacrylate.

Although immobilization of PNPase provides several advantages over the use of free enzyme for synthesis of polyribonucleotides, immobilization on certain supports can present several problems, including loss of enzyme activity upon immobilization and leaching of enzyme from the support under the polymerization conditions. For example, Schnapp et al. Biochemical and Biophysical Research Communications, Vol 70, No. 1 (1976) refers to the problem of leaching of enzyme from CNBr activated Sepharose® and suggests instead the use of amine, hydrazine or hydrazide carrying supports to alleviate the problem.

## SUMMARY OF THE INVENTION

We have discovered that PNPase immobilized on support particles of a solid, water-insoluble epoxy-activated polymer exhibits specific activity in the range of 0.3-0.8 U/g, is leached from the support at a rate < about 5%, generally < 1%, in continuous use over 10 recycles under standard polyribonucleotide synthesis conditions (pH 9, 37 °C), and retains at least 90% of its original activity after being used 10 times for polyribonucleotide synthesis. Specific activity is defined as units of PNPase activity per gram of support (wet weight) .A unit (U) of activity is defined as that activity which will produce 1 µmol of inorganic phosphate in 1 minute under the assay conditions described below. The low leaching rate was unexpected,

because PNPase is a trimer composed of three polypeptide subunits. It is unknown whether all three subunits are covalently attached to the epoxy-activated support particles.

We have also discovered that the yield of the immobilization reaction is strongly dependent on pH of the reaction mixture. PNPase immobilization, as well as immobilization of many other enzymes, ordinarily is carried out at basic pH in the range of about 8 to 10. This corresponds to the range where polyribonucleotide synthesis is conducted. It was previously thought that immobilization should be carried out at approximately the same pH as synthesis in order to avoid changes in PNPase conformation. We have found that, although the immobilization on epoxy-activated support can be carried out anywhere in the range of about pH 4 to 9, yields are significantly better in the range of about pH 4 to 6.

We have also found that the chain length (molecular weight) of polyribonucleotides obtained by immobilized PNPase-catalyzed reactions of nucleoside diphosphates with polyribonucleotides is dependent on the pore size of the support particles, as well as reaction time. Larger pore sizes result in lower molecular weight polyribonucleotides during the early stage (e.g. about the first 30%) of the reaction. Thus, a polyribonucleotide of desired molecular weight range can be obtained by use of a support of proper pore size and proper reaciton time.

## DESCRIPTION OF THE FIGURES

Fig. 1 is a plot showing the effect of pH in the PNPase immobilization reaction.

Fig. 2 is a plot showing the effect of support pore size and reaction time on molecular weight of polyribonucleotide product.

## DETAILED DESCTRIPTION

The epoxy-activated support particles (beads) can be any naturally occurring or synthetic polymer or copolymer that contains or is treated to contain an epoxide (oxirane) group which is accessible to a reactive nucleophilic functional group of a protein, e.g. an amine, sulfhydryl, alcohol, acid, amide, or aromatic carbon group. The epoxy group can be a part of the monomer or comonomer used in synthesis of the polymer, or it can be covalently attached after synthesis by reaction of the polymer with an epoxide-containing compound. Such polymers can be represented as follows:

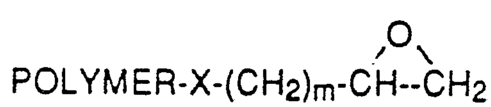

where m is zero or an integer of about 1 to 5 and X is O or S or a group containing N, P, S or C. Examples of suitable naturally occurring polymers include epoxy-activated polysaccharides such as agarose and Sepharose®. Synthetic polymers which can be used include epoxy-activated polyacrylates, polymethacrylates, polyacrylamides, polymethacrylamides, polyvinylacetates, and polyvinyl alcohols. Especially suitable patents 4,070,348, 4,190,713, 4,208,309, 4,247,643 and 4,511,694 Matthews U.S. patents 3,844,892 and 3,932,557, and Bigwood U.S. patents 4,582,860 and 4,612,288, the disclosures of which are incorporated herein. Suitable commercially available materials include VA-Epoxy Biosynth® available from Crescent Chemical Company, Eupergit® C from Roehm Pharma, and epoxy-activated Sepharose® from Pharmacia Laboratories.

Average particle size of the support is not critical, but will generally be in the range of about 1 to 1,000 µm. The particles can be substantially non-porous or can contain pores (channels and cavities) with average diameter in the range of about 0.01 to 1 µm. Particles in the average size range of about 10 to 500 µm containing pores with average diameter in the range of about 0.02 to 0.5 µm are preferred for preparation of polyribonucleotides with chain length in the range of about 200 to 1,000 nucleotides. This is the preferred ssRNA chain length range for producing dsRNA's with higher pharmacological activity and lower toxicity. Most preferred at present are supports with average bead size in the range of about 100 to 300 µm and average pore size in the range of about 0.02 to 0.2 µm.

Immobilization of the PNPase on the epoxy-activated beads is accomplished simply by mixing the

beads with an aqueous buffered solution of PNPase, adjusting to the desired pH by addition of acid, and letting the mixture stand for a few (e.g. 10-40) hours, preferably with mild agitation. As indicated above, the immobilization can be carried out at pH in the range 4 to 9, but pH in the range of 4-6 is preferred for obtaining optimum specific activity and yield. If desired, the beads can be swollen by soaking in buffer before mixing with PNPase. Several buffers were tested for carrying out the immobilization reaction. in the 4-6 pH range. It was found that use of 4-morpholineethanesulfonic acid (MES) buffer resulted in better yields than were obtained with acetate and phosphate buffers.

In the examples below, the following General Methods were used.

## Enzyme Assays

The enzymatic activity of both the soluble and the immobilized form of PNPase were measured by incubating the enzyme (ca. 25 U/mL) in a solution containing tris buffer (0.1 M, pH 9.0), adenosine diphosphate (ADP, 20 mM), $MgCl_2$ (5mM) at 37° C for 15 minutes. The inorganic phosphate produced in the reaction is measured by the method of Bencini et al., Anal. Biochem. Vol. 132, pp. 254-258 (1983). A unit of activity (U) of PNPase is defined as that activity which will produce 1 µmol of inorganic phosphate in 1 minute under the assay conditions.

## HPLC Analysis

HPLC analyses were performed on a Hewlett-Packard 1090 M HPLC equipped with a UV diode array detector and a DuPont GF-250 size exclusion column. The elutant is $Na_2HPO_4$ (0.2M, pH 7.0) at a flow rate of 2 mL/min. The polynucleotides and the nucleoside diphosphates are separated under these conditions.

# EXAMPLES

## Example 1: PNPase Immobilized on Oxirane Acrylic Beads

Eupergit® C 250 L oxirane acrylic beads were swollen in MES buffer (50mL, 0.1 M, pH 5.9) for 15 minutes. The supernatant was decanted and the beads washed with MES buffer (3 X 75 ml). A buffered solution of PNPase (46.5 mL, 3.2 U/mL, tris:HCl (10 mM, pH 8.2), EDTA (1mM), 2- mercaptoethanol (1 mM), glycerol (50% v/v)) was added. (Glycerol is included in commercially available PNPase solution to prevent freezing; EDTA and 2-mercaptoethanol are included as preservatives) The pH of the reaction mixture was adjusted to 5.9 with 1 N HCl and the suspension tumbled slowly for 30h. The beads were allowed to settle and the supernatant decanted. The beads were washed with MES buffer (3 X 5 mL, 0.1 M, pH 5.5) and trishydroxymethylaminomethane (tris) buffer (4 X 5 mL, 0.1 M, pH 8.0). The beads (235 g wet wt.) were found to contain 93.5 U of PNPase activity (62% yield). When stored for one month at 4° C, the immobilized PNPase retained >95% of its original activity. Furthermore, the immobilized PNPase retained >95% of its original activity after being exposed to elevated temperature (37° C for 13 hours). Under the same conditions, the soluble enzyme retains only about 70% of its original activity.

## Example 2: PNPase Immobilized on Epoxy-activated Sepharose® Beads

Epoxy-activated Sepharose® beads (Pharmacia Laboratories, 0.1 g) were swollen in water for 15 minutes and then washed with MES buffer (2 X 1mL, 0.1 M, pH 5.5). The supernatant was decanted and a buffered solution of PNPase (0.25 mL, 3.4 U/mL, tris:HCl (10 mM, pH 8.2), EDTA (1mM), 2-mercaptoethanol (1 mM), glycerol (50% v/v)) was added. The suspension was rocked gently for 24 hours. The beads were washed with MES buffer (3 X 5 mL, 0.1 M, pH 5.5) and tris buffer (3 X 5 mL, 0.1 M, pH 8.0). The beads (0.8 g wet wt.) were found to contain 0.24 U of PNPase activity (30% yield).

## Example 3: PNPase Immobilized on Epoxy-activated Polyvinylalcohol Beads

Epoxy-activated polyvinylalcohol beads (VA-epoxy Biosynth®, Riedel-de Haën, 0.11 g) were swollen in MES buffer (1 mL, 0.1 M, pH 5.9) for 15 minutes. The supernatant was decanted and a buffered solution of PNPase (0.37 mL, 1.2 U/mL, tris:HCl (10 mM, pH 8.2), EDTA (1mM), 2-mercaptoethanol (1 mM), glycerol (50% v/v) was added. The suspension was rocked gently for 24 hours. The beads were allowed to settle and the supernatant was decanted. The beads were washed with MES buffer (3 X 1mL, 0.1 M, pH 5.5) and tris buffer ( 1mL, 0.1 M, pH 8.0) and tris buffer ( 1mL, 1.0 M, pH 9.0). The beads (0.36 g wet wt.) were found to contain 0.24 U of PNPase activity (53% yield).

Example 4: Protein Leaching of PNPase Immobilized on Oxirane Acrylic Beads

PNPase immobilized on Eupergit® C 250 L oxirane acrylic beads (2.16 g gross wet wt., containing 51.7 mg of protein) was added to tris buffer (7.5 mL, 0.1 M, pH 9.0) containing $MgCl_2$ (5 mM) and $Na_2HPO_4$ - (20mL.) The suspension was rocked gently at room temperature. Aliquots (0.1 mL). were removed peridically and assayed for protein by the BCA (bicinchoninic acid) assay method ( Smith, Anal. Biochem. Vol. 150, pp. 76-85. (1985))

| Time (H) | Protein Conc. (ug/mL) | Cumulative Protein Lost (%) |
|---|---|---|
| 0 | 55.4 | 0.80 |
| 1 | 56.5 | 0.81 |
| 2 | 58.6 | 0.83 |
| 4 | 69.8 | 0.97 |
| 24 | 73.9 | 1.01 |
| 48 | 76.2 | 1.03 |
| 72 | 78.3 | 1.04 |
| 144 | 79.9 | 1.05 |
| 168 | 80.1 | 1.05 |

When adjusted for background protein, less than 0.3% of the total protein immobilized was leached from the support after 7 days under reaction conditions.

When adjusted for background protein, less than 0.3% of the total protein immobilized was leached from the support after 7 days under reaction conditions.

Example 4: Multiple Preparation of Polynucleotides with Immobilized PNPase

A suspension of PNPase immobilized on Eupergit® C 250 L oxirane acrylic beads ( 1.9 U, 3.75 g gross wet wt.) in tris buffer (40 mL, 0.1 M, pH 9.0) containing inosine diphosphate (IDP, 20 mM) and $MgCl_2$ (5mM) was rocked gently at 25° C for 42 hours. The immobilized PNPase was filtered and washed with tris buffer (3 X 13.3 mL, 0.1 M, pH 9.0). The combined filtrates were passed through a 0.2 µm filter and placed in a Nucleopore stirred-cell diafiltration appartus equipped with an Amicon PM-10 ultrafilter (10,000 molecular wt. cut-off membrane). The solution was filtered using 60 psi nitrogen gas pressure until the volume of the retentate was <2 mL. The retentate was diluted and then concentrated to a volume of <2 mL sequentially with EDTA solution (80 mL, 0.1 M, pH 7.5), potassium acetate solution (80 mL, 0.1 M, pH 7.5) and water (80 mL). The retentate was dissolved in water (20 mL). Analysis by HPLC indicated the poly inosinic acid had been quantitatively retained in the retentate (50-55 % overall yield) while the nucleoside diphosphate and other low molecular weight compounds were contained in the filtrate. The aqueous solution of polynucleotide is suitable for lyophilization, precipitation or subsequent reaction. The recovered immobilized enzyme retained >90% of its original activity and could be used in 15 additional preparations of polynucleotide.

Other homopolymer and copolymer polyribonucleotides can be produced by the method of this example by substituting the appropriate ribonucleoside diphosphate for the IDP. For example, poly $C_{12}U$ can be made by substituting cytidine diphosphate and uridine diphosphate in the mole ratio 12:1.

Poly I was synthesized as described above using PNPase immobilized on either Eupergit® C or Eupergit® C 250 L (average pore sizes of 30 and 160 nm, respectively). The molecular weight of the poly I produced was measured by size exclusion HPLC. As can be seen in Fig. 2 the reaction catalyzed by

Eupergit® C produces higher molecular weight polymer at conversions of less than 30% than does Eupergit® C 250 L supported enzyme. At conversions greater than 30% Eupergit® C 250 L produces higher average molecular weight polymer than does Eupergit® C.

## Claims

1. Polynucleotide phosphorylase immobilized by covalent bonding to particles of a solid water-insoluble, epoxy-activated organic polymer.

2. Composition of claim 1 wherein the polymer is an epoxy-activated polysaccharide, poly(meth)-acrylate, poly(meth)acrylamide, polyvinylacetate, or polyvinyl alcohol.

3. Composition of claim 2 wherein the polymer is an oxirane acrylic homopolymer or copolymer and wherein the particles are about 1 to 1,000 µm in average diameter and contain pores of average diameter in the range of about 0.01 to 1 µm.

4. Composition of claim 3 wherein the particles are about 10 to 500 µm in average diameter and contain pores of average diameter in the range of about 0.02 to 0.5 µm.

5. Composition of claim 4 wherein the polymer is a copolymer of (A) an ethylenically unsaturated monomer having a glycidyl group, (B) a comonomer having at least two radical polymerizable double bonds, and (C) a radical-polymerizable water soluble comonomer.

6. Composition of claim 5 wherein the polymer is a copolymer of (A) glycidyl methacrylate and/or ally glycidyl ether, (B) N,N′-methylene-bis-methacrylamide, and (C) methacrylamide.

7. Composition of claim 6 wherein the particles are about 100-300 µm in diameter and contain pores about 0.02 to 0.2 µm in diameter.

8. Composition of claim 3 wherein the polymer is an epoxy-activated polyvinylalcohol.

9. A method of immobilizing polynucleotide phosphorylase which comprises reacting it in aqueous solution at a pH in the range of 4 to 6 with particles of a solid water-insoluble, epoxy-activated organic polymer.

10. Method of claim 9 wherein the reaction is conducted in 4-morpholineethanesulfonic acid buffer.

11. Method of claim 10 wherein the polymer is an oxirane acrylic or epoxy-activated polyvinyl alcohol and the particles have an average diameter in the range of about 100-300 µm and pores with an average diameter in the range of about 0.02 to 0.2.

12. In the method of synthesizing polyribonucleotides from nucleoside diphosphates in the presence of immobilized polynucleotide phosphorylase, the improvement which comprises using polyribonucleotide phosphorylase immobilized by covalent bonding to particles of a solid water-insoluble, epoxy-activated organic polymer.

13. Improvement of claim 12 wherein the polymer is an epoxy-activated polysaccharide, poly(meth)-acrylate, poly(meth)acrylamide, polyvinylacetate, or polyvinyl alcohol.

14. Improvement of claim 13 wherein the polymer is an oxirane acrylic homopolymer or copolymer and wherein the particles are about 1 to 1,000 µm in average diameter and contain pores of average diameter in the range of about 0.01 to 1 µm.

15. Improvement of claim 14 wherein the particles are about 10 to 500 µm in average diameter and contain pores of average diameter in the range of about 0.02 to 0.5 µm.

16.Improvement of claim 15 wherein the polymer is a copolymer of (A) an ethylenically unsaturated monomer having a glycidyl group, (B) a comonomer having at least two radical polymerizable double bonds, and (C) a radical-polymerizable water soluble comonomer.

17. Improvement of claim 16 wherein the polymer is a copolymer of (A) glycidyl methacrylate and/or allyl glycidyl ether, (B) N,N′-methylene-bis-methacrylamide, and (C) methacrylamide.

18. Improvement of claim 17 wherein the particles are about 100-300 µm in diameter and contain pores about 0.02 to 0.2 µm in diameter.

19. In the method of producing ribonucleotide diphosphates by phosphorolysis of polynucleotides in presence of immobilized polynucleotide phosphorylase, the improvement which comprises using polynucleotide phosphorylase immobilized by covalent bonding to particles of a solid water-insoluble, epoxy-activated organic polymer.

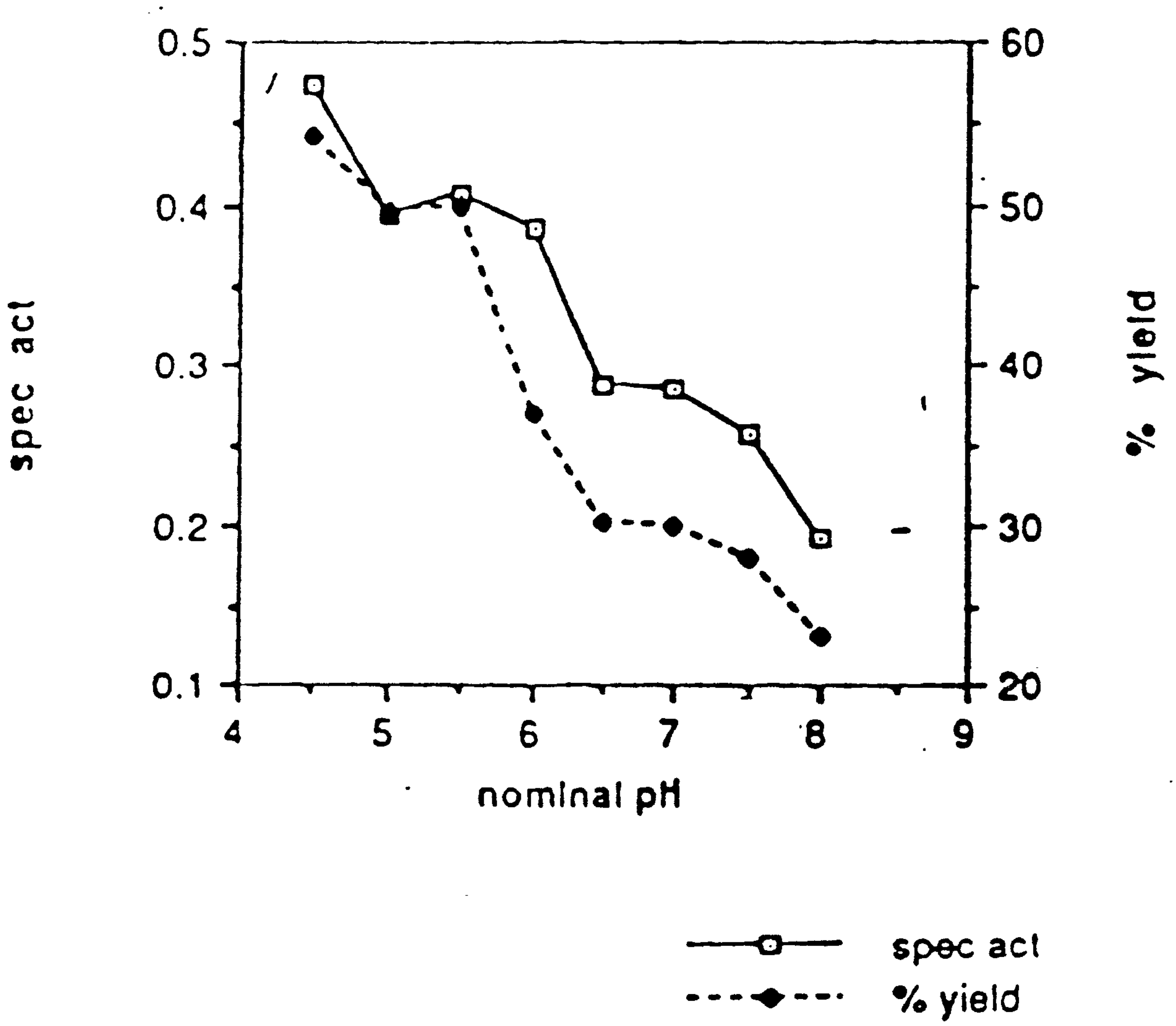
0.5
0.4
0.3
0.2
0.1
60
50
40
30
20
4
5
6
7
8
9
spec act
% yield
nominal pH
spec act
% yield

FIG. 1

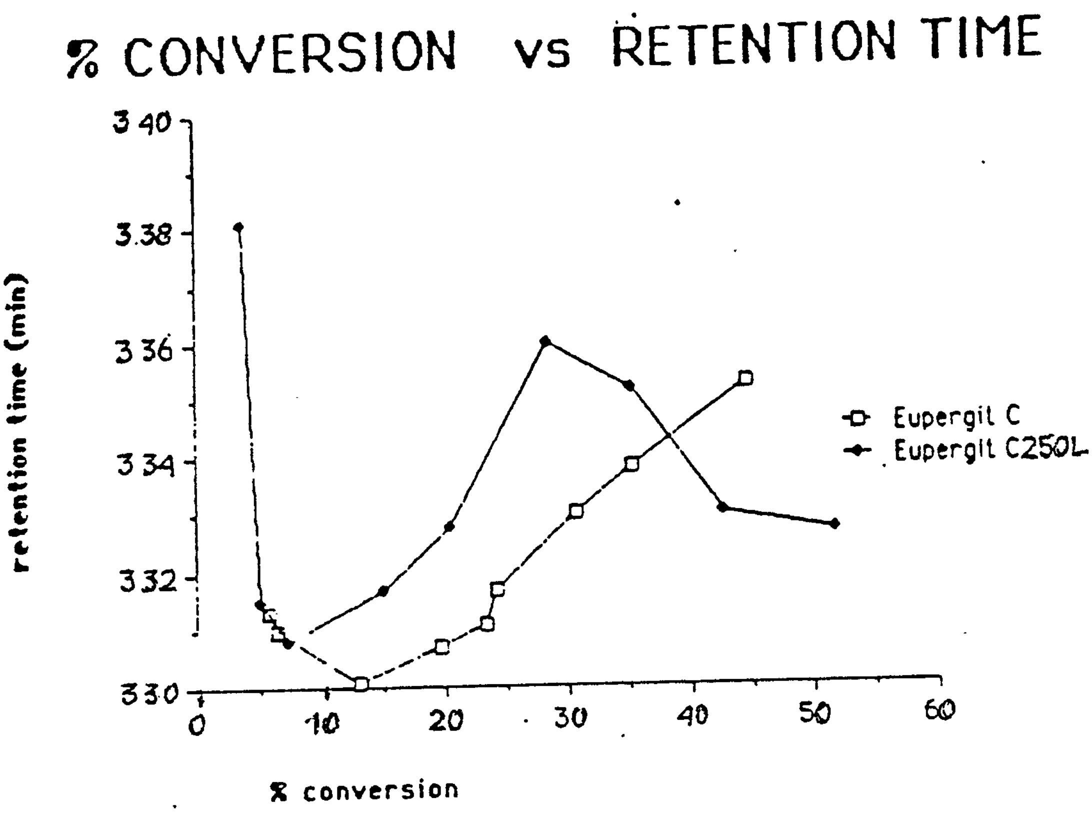
% CONVERSION vs RETENTION TIME
3.40
3.38
3.36
3.34
3.32
3.30
retention time (min)
0
10
20
30
40
50
60
% conversion
Eupergit C
Eupergit C250L

FIG. 2